# Europäisches Patentamt

## European Patent Office

## Office européen des brevets

(19)

(11) Veröffentlichungsnummer: **0 113 416**
**B1**

(12) **EUROPÄISCHE PATENTSCHRIFT**

(45) Veröffentlichungstag der Patentschrift:
**29.01.86**

(51) Int. Cl.⁴: **C 07 D 213/803**

(21) Anmeldenummer: **83111705.6**

(22) Anmeldetag: **23.11.83**

(54) Verfahren zur Herstellung von Nicotinsäureamide.

(30) Priorität: **02.12.82 DE 3244522**

(43) Veröffentlichungstag der Anmeldung:
**18.07.84 Patentblatt 84/29**

(45) Bekanntmachung des Hinweises auf die Patenterteilung:
**29.01.86 Patentblatt 86/5**

(84) Benannte Vertragsstaaten:
**AT BE CH DE FR GB IT LI NL**

(56) Entgegenhaltungen:
**GB - A - 1 078 663**
**US - A - 2 412 749**
**US - A - 2 427 400**

(73) Patentinhaber: **BASF Aktiengesellschaft,**
**Carl-Bosch-Strasse 38, D-6700 Ludwigshafen (DE)**

(72) Erfinder: **Baur, Karl Gerhard, Dr.,**
**van-Leyden-Strasse 17, D-6700 Ludwigshafen (DE)**
Erfinder: **Diehl, Volker, Dr., Haardtstrasse,**
**D-6701 Ellerstadt (DE)**
Erfinder: **Stops, Peter, Madenburgstrasse 3,**
**D-6701 Altrip (DE)**
Erfinder: **Hellbach, Hans, Stettiner Strasse 14,**
**D-6840 Lampertheim (DE)**
Erfinder: **Brunner, Erwin, Dr., Neuhausener Weg 1,**
**D-6700 Ludwigshafen 29 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von Nicotinsäureamid, auch Vitamin PP und Niacinamid genannt, durch Amidierung einer überwiegend Nicotinsäure enthaltenden Nicotinsäure/Nicotinsäureamid-Schmelze.

Die Herstellung von Nicotinsäureamid durch Umsetzung von Nicotinsäure mit gasförmigem Ammoniak bei erhöhter Temperatur unter Abdestillieren des Reaktionswassers ist aus der US-Patentschrift Nr. 2412749 bekannt. Dieses Verfahren ist unbefriedigend, da die Ausbeute nur 60% d.Th. beträgt und eine mehrstufige Kristallisation zur Reinigung des Nicotinsäureamids erforderlich ist, so dass die Ausbeute an reinem Amid nochmals geringer ist.

Es war daher das Ziel der Erfindung, ein verbessertes Verfahren zur Herstellung von Nicotinsäureamid vorzuschlagen, bei dem die Amidierungsreaktion mit besserer Ausbeute verläuft und das keiner Kristallisation bedarf.

Diese Aufgabe wurde mit der Umsetzung einer Schmelze aus Nicotinsäure und Nicotinsäureamid mit Ammoniak und Vakuumdestillation des erhaltenen Amidierungsgemisches gelöst.

Es wurde nämlich überraschenderweise gefunden, dass man in einfacher Weise mit guten Ausbeuten und in guter Reinheit Nicotinsäureamid erhält, wenn man in einer ersten Stufe eine Lösung von Nicotinsäure in Nicotinsäureamid bei Temperaturen von 190 bis 240° C mit einem Überschuss Ammoniak unter gleichzeitigem Abdestillieren des Reaktionswassers umsetzt, die erhaltene Lösung aus Nicotinsäure und überwiegend Nicotinsäureamid in einer zweiten Stufe einer Vakuumdestillation unterwirft, in der reines Nicotinsäureamid am Fuss der Kolonne und eine Mischung von Nicotinsäure und Nicotinsäureamid, mit einem auf den Zulauf bezogenen niedrigeren Nicotinsäureamidgehalt am Kopf abgezogen wird, und wobei man die in der zweiten Stufe erhaltene Nicotinsäure/Nicotinsäureamidmischung in die erste Stufe zurückführt und mit frischer Nicotinsäure ergänzt.

Die Umsetzung in der ersten Stufe erfolgt bei Normaldruck oder bei Überdruck bis zu etwa 60 bar. Die Reaktionstemperatur beträgt 190 bis 240° C, normalerweise 200 bis 220° C.

Man wendet dabei zweckmässig einen Überschuss an Ammoniak, bezogen auf eingesetzte Nicotinsäure, an. Der molare Überschuss beträgt z.B. die 0,5- bis 4-, insbesondere die 1- bis 2,5-molare Menge über die zum Umsatz benötigte Menge hinaus.

Führt man die Amidierung drucklos aus, so erhält man bei Verweilzeiten von mehreren Stunden einen Umsatz bis zu 85%, bei erhöhtem Druck, z.B. 2 bis 60 bar, einen Umsatz bis zu 95%.

Als nicht mehr verwertbare Nebenprodukte entstehen geringe Mengen Pyridin und Nicotinsäurenitril, die mit dem Reaktionswasser abdestilliert werden.

Das für die Amidierung verwendete Ausgangsgemisch aus Nicotinsäure und Nicotinsäureamid soll so zusammengesetzt sein, dass es bei der Reaktionstemperatur vollständig flüssig und homogen ist.

Zweckmässig wählt man solche Mischungen, bei denen der Nicotinsäureanteil überwiegt, z.B. mit einem Gehalt an 50 bis 80% Nicotinsäure. Diese Mischungen haben Schmelztemperaturen zwischen 180 bis 210° C. Die Schmelztemperatur der reinen Nicotinsäure von 236° C, bei der schon die Zersetzung der Nicotinsäure beginnt, braucht so nicht erreicht oder überschritten zu werden.

Grundsätzlich könnten auch Mischungen aus Nicotinsäure/Nicotinsäureamid, in denen das Amid überwiegt, amidiert werden, wenn man nur in dem Masse frische Nicotinsäure oder rückgeführtes Nicotinsäure enthaltendes Kopfprodukt der 2. Stufe zugibt, wie die Amidierung erfolgt. Dies bietet jedoch kaum Vorteile, da dann der Reaktionsraum vergrössert werden muss.

Die Amidierungsreaktion kann diskontinuierlich in einem Rührkessel oder Autoklaven oder vorzugsweise kontinuierlich in an sich bekannten Anordnungen wie Rührkesselkaskade, Blasensäule, Druckrohr oder Schlaufenreaktorkaskade ausgeführt werden.

Die Zusammensetzung des in der Amidierungsreaktion erhaltenen Rohgemisches variiert in Abhängigkeit von den Reaktionsparametern Druck, Verweilzeit, Temperatur und Ammoniak-Überschuss und beträgt z.B. zwischen 60 und 95 Gew.% Nicotinsäureamid, 39 und 4 Gew.% Nicotinsäure, 0,1 bis 2 Gew.% Nicotinsäurenitril und Spuren Pyridin, Wasser und Ammoniak.

Die zweite Stufe des erfindungsgemässen Verfahrens, die Destillation des Amidierungsgemisches, erfüllt zwei Aufgaben, nämlich die Reinigung des rohen Nicotinsäureamids und die Rückführung der nicht umgesetzten Nicotinsäure in die Amidierungsstufe. Die Destillation ist gewissen Rahmenbedingungen unterworfen. Physikalische Messungen zeigen zwar, dass entsprechend der Dampfdruckkurven der reinen Verbindungen Nicotinsäure die höhersiedende Komponente im Vergleich zum Nicotinsäureamid ist. Das tatsächliche Destillationsverhalten des Nicotinsäure/Nicotinsäureamid-Gemisches zeigt jedoch, dass sich ein Gemisch mit bis zu 50 Gew.% Nicotinsäure wie die leichterflüchtige Komponente verhält. Somit ist es möglich, die Nicotinsäure am Kopf der Destillationskolonne anzureichern und reines Nicotinsäureamid im Sumpf zu erhalten.

Da Nicotinsäureamid sich oberhalb 190° C in Abhängigkeit von der Verweilzeit und der Temperatur zersetzt, wird die Destillation zweckmässig bei gutem Vakuum am Kopf der Kolonne (2-10 mbar) durchgeführt. Kolonne und Verdampfer sollten dementsprechend nur einen sehr geringen Druckverlust verursachen. Man verwendet deshalb vorzugsweise Kolonnenpackungen, die eine geordnete, sich stetig in allen Raumrichtungen wiederholende Hohlstruktur aufweisen unter Vermeidung senkrechter Hohlkanäle im Innern der Kolonnenpackung und unter Gewährleistung dichten Abschliessens der Strukturelemente an den Kolonnenmantel. Als Verdampfer kommen beispielsweise Dünnschicht- oder Fallfilmverdampfer in · Betracht, wobei neben geringem Druckverlust ein möglichst geringer hold-up im Verdampfungsraum gefordert ist.

Da das Ziel der erfindungsgemässen Destillationsstufe vorwiegend die Abreicherung des Gemisches an Nicotinsäure ist, sollte die Kolonne über eine hinreichende Trennstufenzahl im Abtriebsteil verfügen. Der Verstärkungsteil der Kolonne ist dagegen möglichst klein ausgelegt, um eine zu starke Anreicherung von Nicotinsäure am Kopf der Kolonne zu verhindern. Andernfalls übersteigt die Kondensationstemperatur die Schmelztemperatur des Gemisches am Kopf der Kolonne.

Das reine Nicotinsäureamid wird am Sumpf der Kolonne, gegebenenfalls an einem Seitenabzug oberhalb des Sumpfes, abgezogen.

Am Kopf der Kolonne erhält man Nicotinsäure zusammen mit Nicotinsäureamid und den anderen niedrig siedenden Komponenten Nicotinsäurenitril und Pyridin. Eine Gewinnung von reiner Nicotinsäure am Kopf der Kolonne ist wegen der bereits geschilderten Azeotropie nicht möglich. Der Schmelzpunkt des azeotropen Gemisches liegt mit ca. 210° C über der Siedetemperatur des Gemisches, so dass es notwendig wäre, am Kopf der Kolonne ein Lösungsmittel mitzuführen. Da ein systemfremdes Lösungsmittel dem Zulaufgemisch vor dem Einbringen in die Kolonne zugegeben und nach erfolgter Destillation aus dem Rückführungsstrom wieder entfernt werden müsste, wird, um dies zu vermeiden, als Lösungsmittel vorzugsweise Nicotinsäureamid verwendet. Die gewünschte Menge an Nicotinsäureamid, d.i. ca. 60 Gew.%, lässt sich durch Auslegung des Verstärkungsteils der Kolonne und Einstellung des Kolonnenrücklaufverhältnisses steuern.

Einen Einfluss auf das Siede-/Schmelzverhalten am Kopf der Kolonne übt darüber hinaus die Konzentration an niedrigsiedenden Komponenten aus, insbesondere die Anwesenheit von Nicotinsäurenitril. Schon geringe Anteile dieser leichter flüchtigen Komponenten erniedrigen die Siedetemperatur bei konstantem Druck stark. Andererseits ist die Gefrierpunktserniedrigung, die durch diese leichter flüchtigen Komponenten verursacht wird, vernachlässigbar. Dies bedeutet, dass man zweckmässig die Nicotinsäurenitril-Konzentration sowie die Konzentration an allen anderen niedrigsiedenden Verbindungen wie z.B. Pyridin, Wasser und Ammoniak im Zulauf so gering wie möglich hält.

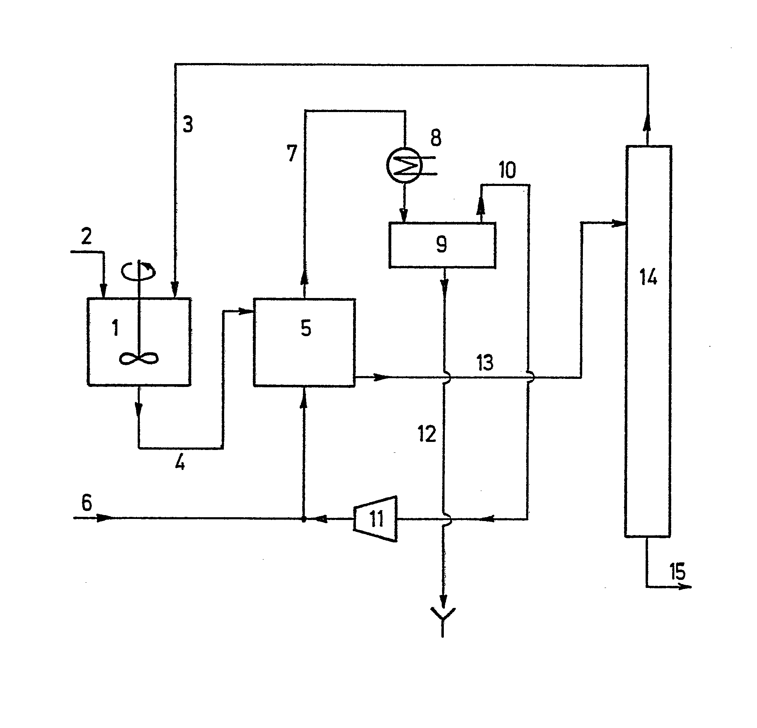

Zweckmässig führt man die Destillation und das gesamte Verfahren kontinuierlich aus, wobei Amidierung und Destillationsstufe gekoppelt sind. Dies wird auch anhand der Figur 1 und dem folgenden Beispiel näher erläutert.

Einem Mischkessel 1 wurden stündlich 110 kg frische Nicotinsäure über Leitung 2 und 149 kg/h des bei der Destillation in Kolonne 14 als Kopfprodukt erhaltene Nicotinsäure/Nicotinsäureamid-Gemisch (Gewichtsverhältnis 40:60) über Leitung 3 zugeführt. Über Leitung 4 wurde das Gemisch dem Reaktor 5 zugeleitet, in den über Leitung 6 gasförmiges Ammoniak eingegeben wurde. Ein Teil des im Reaktor 5 benötigten Ammoniaks wurde aus dem Dampf/Flüssigkeits-Abscheider 9 über Leitung 10 dem Kompressor 11 zugeführt und in die Ammoniak-Versorgung eingespeist. Aus dem Reaktor 5 wurde über Leitung 7 überschüssiges Ammoniak, das während der Reaktion gebildete Wasser und der Anteil an mitgeführten organischen Produkten wie Nicotinsäureamid, Nicotinsäure, Pyridin und Nicotinsäurenitril, ständig abgezogen. Nach Abkühlen dieses Stromes im Wärmetauscher 8 wurde das Gemisch in den Flüssig/Dampf-Abscheider 9 eingeleitet. Gasförmiges Ammoniak wurde über Leitung 10 zum Reaktor zurückgeführt und die wässerige Lösung aus dem Abscheider wurde über Leitung 12 entsorgt.

Die im Reaktor erhaltene Schmelze von rohem Nicotinsäureamid wurde über Leitung 13 in den oberen Teil einer Rektifikationskolonne 14 eingebracht. Am Sumpf der Kolonne wurde über Leitung 15 in einem Seitenabzug 100 kg/h reines, geschmolzenes Nicotinsäureamid erhalten. Am Kopf der Kolonne wurde ein Kreislaufstrom bestehend aus 39 Gew.% Nicotinsäure, 60 Gew.% Nicotinsäureamid und wenig Nicotinsäurenitril isoliert und über Leitung 3 in den Mischbehälter 1 zurückgeführt.

## Patentansprüche

1. Verfahren zur Herstellung von Nicotinsäureamid durch Amidierung von Nicotinsäure, dadurch gekennzeichnet, dass man in einer ersten Stufe eine Lösung von Nicotinsäure in Nicotinsäureamid bei Temperaturen von 190 bis 240° C mit einem Überschuss Ammoniak unter gleichzeitigem Abdestillieren des Reaktionswassers umsetzt, die erhaltene Lösung aus Nicotinsäure und überwiegend Nicotinsäureamid in einer zweiten Stufe einer Vakuumdestillation unterwirft, in der reines Nicotinsäureamid am Fuss der Kolonne und eine Mischung von Nicotinsäure und Nicotinsäureamid, die bezogen auf den Zulauf an Nicotisäureamid abgereichert ist, am Kopf abgezogen wird, und wobei man die in der zweiten Stufe erhaltene Nicotinsäure/Nicotinsäureamidmischung in die erste Stufe zurückführt und mit frischer Nicotinsäure ergänzt.

2. Verfahren gemäss Anspruch 1, dadurch gekennzeichnet, dass man die Amidierung bei Temperaturen von 200 bis 220° C kontinuierlich durchführt.

3. Verfahren gemäss Anspruch 1, dadurch gekennzeichnet, dass man zur Amidierung eine Lösung Nicotinsäure in Nicotinsäureamid mit einem Gehalt von 50 bis 80 Gew.% Nicotinsäure, bezogen auf die Lösung verwendet.

4. Verfahren gemäss Anspruch 1, dadurch gekennzeichnet, dass man aus der ersten Stufe eine Mischung von Nicotinsäure mit Nicotinsäureamid mit einem Gehalt von 60 bis 95 Gew.% Amid abzieht und in die Destillationsstufe überführt.

5. Verfahren gemäss Anspruch 1, dadurch gekennzeichnet, dass man die Destillation der 2. Stufe kontinuerlich betreibt und am Kopf ein Gemisch aus ungefähr gleichen Mengen Nicotinsäure und Nicotinsäureamid abzieht.

## Claims

1. A process for the preparation of nicotinamide by amidation of nicotinic acid, wherein a solution of nicotinic acid in nicotinamide is reacted in a first stage with an excess of ammonia at from 190 to 240° C, the water of reaction being removed at the same time by distillation, the resulting solution of nicotinic acid and, predominantly, nicotinamide is subjected, in a second stage, to vacuum distillation in which pure nicotinamide is removed at the bottom of the column and a mixture of nicotinic acid and nicotinamide with a lower nicotinamide content than the feed is removed at the top of the column, and the nicotinic acid/nicotinamide mixture obtained in the second stage is recycled to the first stage and replenished with fresh nicotinic acid.

2. A process as claimed in Claim 1, wherein the amidation is carried out continuously at from 200 to 220° C.

3. A process as claimed in Claim 1, wherein a solution of nicotinic acid in nicotinamide with a nicotinic acid contend of 50 to 80% by weight, based on the solution, is used for the amidation.

4. A process as claimed in Claim 1, wherein a mixture of nicotinic acid and nicotinamide with a nicotinamide content of from 60 to 95% by weight is removed from the first stage and transferred to the distillation stage.

5. A process as claimed in Claim 1, wherein the distillation in the 2nd stage is carried out continuously, and a mixture of approximately equal amounts of nicotinic acid and nicotinamide is removed at the top.

## Revendications

1. Procédé de préparation de l'amide de l'acide nicotinique par amidation de l'acide nicotinique, caractérisé en ce que, dans une première phase, on transforme l'acide nicotinique en solution, à des températures de 190 à 240° C, par de l'ammoniac en excès en amide nicotinique, l'eau de réaction étant simultanément séparée par distillation, puis on soumet la solution obtenue, qui contient de l'acide nicotinique et de façon prépondérante de l'amide nicotinique, dans une deuxième phase à une distillation sous vide dans une colonne, au pied de laquelle on soutire de l'amide nicotinique pur et au sommet de laquelle on obtient un mélange d'acide nicotinique et d'amide nicotinique, plus riche en amide nicotinique que la solution de départ, le mélange d'acide nicotinique et d'amide nicotinique obtenu dans la deuxième phase étant recyclé dans la première phase après addition d'acide nicotinique frais.

2. Procédé suivant la revendication 1, caractérisé en ce que l'amidation est réalisée en continu entre 200 et 220° C.

3. Procédé suivant la revendication 1, caractérisé en ce que l'on effectue l'amidation avec une solution d'acide nicotinique dans l'amide nicotinique d'une teneur en acide nicotinique comprise entre 50 et 80% du poids de la solution.

4. Procédé suivant la revendication 1, caractérisé en ce que l'on soutire de la première phase un mélange d'acide nicotinique et d'amide nicotinique d'une teneur en amide comprise entre 60 et 95% en poids, qui est amené dans la phase de distillation.

5. Procédé suivant la revendication 1, caractérisé en ce que la distillation de la deuxième phase est réalisée en continu et le mélange soutiré en tête de la colonne contient des proportions approximativement identiques d'acide nicotinique et d'amide nicotinique.